# EUROPEAN PATENT SPECIFICATION

(11) **EP 2 024 013 B1**
(45) Date of publication and mention of the grant of the patent: **11.04.2012**
(21) Application number: 07761615.9
(22) Date of filing: 30.04.2007
(51) Int. Cl.: A61N 1/05

(54) **IMPLANTABLE MEDICAL LEAD ASSEMBLIES WITH DELIVERY TETHER**
IMPLANTIERBARE MEDIZINISCHE ELEKTRODENANORDNUNGEN MIT FREISETZUNGSHALTEGURT
ENSEMBLES DE FILS ELECTRIQUES MEDICAUX IMPLANTABLES COMPORTANT UNE ATTACHE DE DISTRIBUTION

(30) Priority: 28.04.2006 US 413316
(43) Date of publication of application: 18.02.2009
(73) Proprietor: Medtronic, Inc., Minneapolis MN 55432-5604 (US)
(72) Inventor: CROSS, Thomas E., Jr., St. Francis, MN 55070 (US); WILLIAMS, Michaelene M., Fridley, MN 55432 (US)
(74) Representative: Hughes, Andrea Michelle
(86) International application number: PCT/US2007/067840
(87) International publication number: WO 2007/128002

(56) References cited:
- EP-A2- 0 657 185
- US-A- 5 086 787
- US-A1- 2005 033 394
- US-B1- 6 434 431

## Description

### Background

The present invention relates to implantable medical leads for connection between a stimulating control device and one or more stimulation or sensing electrodes and methods of manufacturing such leads, and more particularly to implantable leads and lead assemblies with coiled electrodes and means for implanting the same.

Systems and methods for electrical stimulation of electrically excitable tissue within the body of a living subject have been developed utilizing stimulating electrodes and a signal generator or control device to supply electrical charges in a controlled or predetermined manner. Such systems and methods have been developed specifically based upon a desired condition, such as to alleviate pain or to stimulate muscle movement, and based upon the application with a subject's body. For bodily applications where alleviation of pain is the goal, one or more stimulating and/or sensing electrodes can be implanted within nerve tissue, the brain or spinal cord for blocking pain sensation by electrical stimulation. For muscle tissue stimulation, a stimulating electrode can be implanted in a muscle tissue, whereby electrical current that is typically provided as pulses can cause muscle tissue reaction that may be controlled to cause movement of a subject's body part. Sensing electrodes are used for determining actions of the body.

Signal generators can determine when, how long, and/or the amperage of current pulses that are to be applied for the specific application, and often include hard-wired circuitry, a microprocessor with software and/or embedded logic as the controlling system for determining and dictating current pulses. Such signal generators may also be implanted within the subject's body, and typically such an implantation is done to position the signal generator close to the stimulating and/or sensing electrodes, with interconnecting medical leads for conducting current pulses to and from the stimulating and sensing electrodes. Implantable medical leads and externally utilized leads for these purposes are typically insulated conductors or conductive elements (e.g., a conductor disposed within a lead body), with conductive terminations at both ends for electrical connection with the signal generator and one or more electrodes. Implantable medical leads further have requirements for safe interbody use such as tissue compatibility, surgical procedure dynamics, and body fluid accommodation.

Signal generation and muscle tissue stimulation systems have more recently been envisioned for more complex control of a subject's bodily actions. One particularly complex muscular control concept has recently been considered for the purpose of re-teaching a subject how to swallow, the condition of inability to swallow being known as dysphagia. Techniques and methods of stimulating muscles within the neck region of a patient for the purposes of causing specifically determined muscles to react as a swallowing effect are described in PCT Publication No. WO 2004/028433, having a publication date of April 8, 2004. Specifically, by implanting electrodes in two or more muscles of the upper airway musculature and connecting the electrodes with a signal generator that provides coordinated control signals, a swallowing action can be induced in the patient. Other specific techniques and methods are also disclosed in U.S. Patent Nos. 5,725,564; 5,891,185; 5,987,359; 6,104,958; and 6,198,970; all to Freed et al. Other techniques and methods are disclosed in U.S. Patent Application Serial No. 11/611,365, filed December 15, 2006, and entitled "Method and Apparatus for Assisting Deglutition."

For these and a variety of other implanted electrode stimulation treatments, conventional leads may not be optimal. For example, it may be difficult at best to achieve long term fixation of a medical lead's electrode at or against certain target tissue sites when employing conventional electrode configurations. Some muscles/muscle tissues are one such example whereby suturing or otherwise affixing a flat or ring-type electrode to the muscle's surface may not maintain a long term connection. Similarly, other target tissue sites are sensitive/fragile, or may require a more intimate contact with the electrode(s) to achieve the desired application of electrical impulses thereto via the electrode(s). Even further, muscles (as well as some other targeted tissue) will repeatedly move (e.g., contract) over time; the electrode used under these circumstances preferably exhibits some extensibility to accommodate these movements. Unfortunately, conventional flat or ring-type electrodes may not be able to satisfy these constraints.

To better meet the above needs (as well as possibly other needs) presented by some surgical sites, a coiled electrode can be employed. Coiled electrodes are generally known, can provide a form of self-fixation to the target tissue, ensure intimate contact with the tissue in question, and can be naturally extensible. For certain applications, it is desirable that the selected coiled electrode be soft or pliable so as to exhibit desired flexibility and/or minimize possible tissue damage. Coiled electrodes formed of platinum and iridium are an example of an acceptable coiled electrode material construction. With these and other similar coiled electrodes, the implantation technique generally entails mounting the coiled electrode to the conductor of the medical lead, delivering the medical lead to the target site, and then inserting the coiled electrode into the target tissue. As part of this insertion, a pulling force is applied to the distal end of the coiled electrode in piercing or otherwise inserting the coiled electrode into the target tissue. Due to the soft nature of many coiled electrodes, this pulling force can cause the coiled electrode to overtly stretch in a relatively inelastic manner, thus possibly damaging the coiled electrode. US 5,086,787 discloses an implantable stimulation lead including a spacer coil wound around a suture thread.

In light of the above, a need exists for a coiled electrode lead assembly configuration that promotes insertion of the coiled electrode into targeted tissue without overtly stretching the coiled electrode.

### Summary

The invention is defined in claims 1 and 10.

In a method of implanting a medical lead using an embodiment of the invention, the coiled electrode is coupled to the
conductor. The tether line is coupled to the coiled electrode at a point proximal a distal end of the electrode, and a leading segment of the tether line extends distal the distal end. The needle is coupled to the leading segment. The lead body is then advanced toward a target site. Target tissue is pierced by the needle. A pulling force is applied to the needle to draw the coiled electrode into contact with the target tissue. In this regard, the pulling force is transferred by the tether line on to the coiled electrode at a point proximally spaced from the distal end of the coiled electrode. As a result, the pulling force is transferred into a pushing force as applied to the coiled electrode. The tether line is then severed, and the needle removed from the target site.

### Brief Description of the Drawings

FIG. 1 is a side view of an implantable medical lead assembly in accordance with aspects of the present disclosure;

FIG. 2 is a cross-sectional view of a portion of the lead assembly of FIG. 1;

FIG. 3 is a side view of a coiled electrode useful with the assembly of FIG. 1;

FIG. 4 is an exploded side view of a tether line and a coiled electrode useful with the assembly of FIG. 1;

FIG. 5A is a side view of the tether line and coiled electrode of FIG. 4 upon final assembly;

FIG. 5B is a cross-sectional view of the assembly of FIG. 5A;

FIG. 6 is a partial cross-sectional view of a portion of the assembly of FIG. 1; and

FIG. 7 is a side view of another embodiment lead assembly in accordance with aspects of the present disclosure.

### Detailed Description

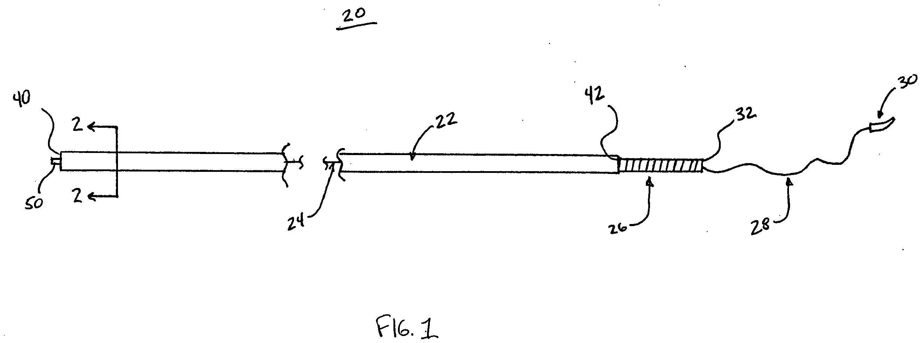

One embodiment of an implantable medical lead assembly 20 in accordance with aspects of the present disclosure is shown in FIG. 1. The lead assembly 20 includes a lead body 22, an elongated conductor 24, a coiled electrode 26, a tether line 28 and an optional needle 30. Details on the various components are provided below. In general terms, however, the conductor 24 is maintained by (e.g., electrically insulated) the lead body 22, and is coupled to the coiled electrode 26. The tether line 28 is also coupled to the coiled electrode 26, with a segment of the tether line 28 extending distal the electrode 26. In this regard, an initial point of coupling between the electrode 26 and the tether line 28 is proximal a distal end 32 of the electrode. Finally, where provided, the needle 30 is connected to the tether line 28 opposite the coiled electrode 26. With this configuration, a pulling force applied to the tether line 28, for example via a pulling force imparted upon the needle 30 during an implantation procedure, is transferred to the coiled electrode 26 proximal the distal end 32. Effectively, then, the pulling force is transferred to a pushing force upon the electrode 26, thereby reducing possible stretching of the coiled electrode 26 during implantation. Lead assemblies (or, more simply, leads) incorporating the above features can include additional components and/or provide desired characteristics appropriate for a particular end use application, and are implantable at a number of different bodily regions and to a variety of different target tissues.

The lead body 22 can assume a variety of forms as known in the implantable medical lead art, useful in electrically insulating the conductor 24. The lead body 22 can comprise any number of layers, which layers may be located directly on the conductor 24 or spaced from the conductor 24, and may include any number of functional layers. In some embodiments, the lead body 22 is formed of a silicone rubber material, although other materials selected to exhibit one or more properties desired for a particular implant application or procedure (e.g., softness, lubricity, etc.) are equally acceptable.

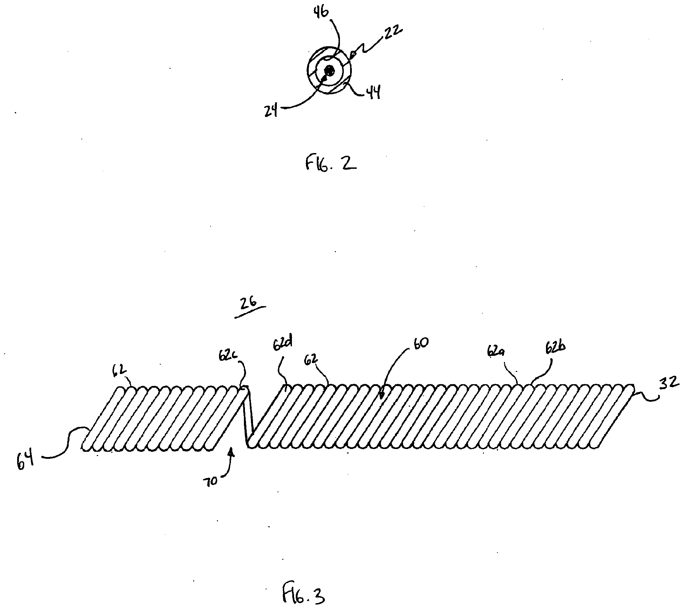

The lead body 22 can be described as extending between or defining a proximal side 40 and a distal side 42. While the lead body 22 is generally illustrated as having a continuous construction, in some embodiments the lead body 22 can be comprised of two or more sections having differing characteristics (either integrally formed, or separately provided and subsequently assembled). For example, the lead body 22 can have a decreased or decreasing diameter adjacent the distal side 42 as compared with a diameter adjacent the proximal side 40. With additional reference to FIG. 2, in some embodiments the lead body 22 is tubular, having a side wall 44 defining a lumen 46. As shown, the lumen 46 is sized to receive the conductor 24. Alternatively, portions of the lead body 22 can have a more solid construction (e.g., akin to a solid shaft) in maintaining the conductor 24 as described below. In connection with either of these constructions, however, the lead body 22 provides or forms the lumen 46 at or adjacent the distal side 42 for co-axially receiving the coiled electrode 26 as described below. In yet other embodiments, however, an entirety of the lead body 22 is solid and does not provide or form a discernable lumen. Finally, while the lead assembly 20 is depicted as including the single lead body 22, two or more of the lead bodies 22 can be provided and connected (e.g., bonded) to each other.

The conductor 24 can extend a substantial length of the lead body 22, forming or defining a proximal lead termination 50 that is electrically coupleable to one or more components of an implantable electrical stimulation and/or sensing system (e.g., lead extender, stimulation control unit or generator, etc.). A distal lead termination (hidden in FIG. 1) may also be formed, and is adapted for electrical coupling to the coiled electrode 26 as described below. In this regard, the conductor 24 can comprise any known or developed conductive wire or the like that may be a solid element (e.g., shaft, coil, etc.), and/or be comprised as a stranded conductor as such are well-known. Stranded wire as used for the conductor 24 would typically be more flexible as compared with solid wire. However, a solid wire is typically more capable of being deformed to hold a shape, and can exhibit a spring-back characteristic that may be useful with leads in accordance with some embodiments of the present disclosure. The lead termination 50 may be merely uninsulated wire portions for connection with other electrical connectors, or may comprise the connectors themselves as fixed to the end(s) of the conductor 26.

While FIGS. 1 and 2 reflects the single conductor 24 within the lead body 22, in other embodiments, two or more of the conductors 24 can be provided, and can be insulated from one another in a conventional manner (e.g., by insulation material coating). Further, while the lead body 22 is illustrated in FIG. 2 as forming or defining the continuous lumen 46, in other embodiments, the lumen 46 can be discontinuous, or the lead body 22 need not form or define the lumen 46. For example, the lead body 22 can encompass the conductor 26 within a material thickness of the lead body 22 (e.g., the lead body 22 can be molded to the conductor 26 that otherwise is provided in coil form, etc.).

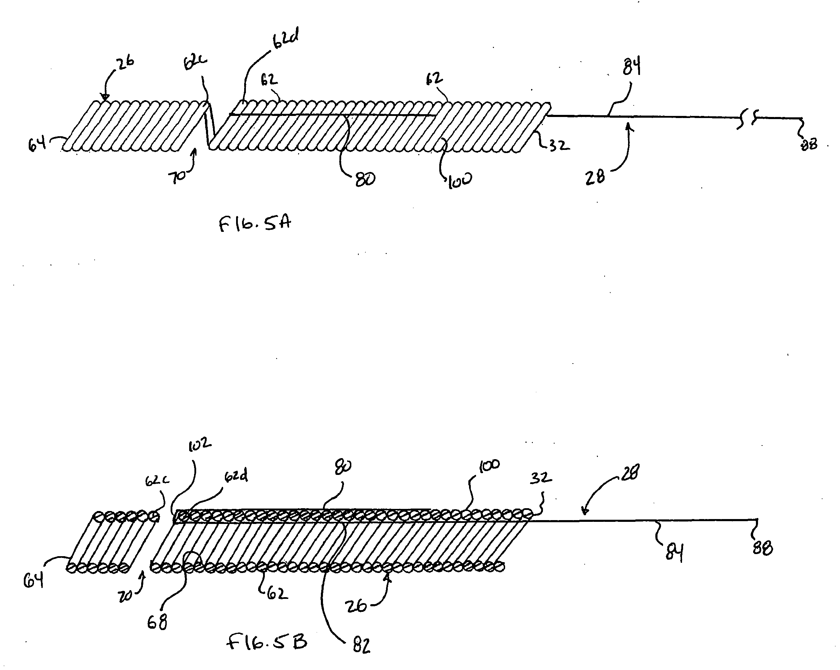

With reference to FIGS. 1 and 3, the coiled electrode 26 can assume a variety of forms, and in some embodiments is formed of soft, surgically safe material. In one such embodiment, the coiled electrode 26 is a platinum iridium electrode. Regardless, the coiled electrode 26 can be a wire 60 (or a plurality of wires) wound to form a plurality of adjacent turns or coils 62 that combine to define a proximal end 64 (FIG. 3), the distal end 32, and a central passageway (hidden in the views of FIGS. 1 and 3, but shown at 68 in FIGS. 5A and 5B). The coiled electrode 26 can be tightly wound such that a majority of adjacent ones of the turns 62, such as the first and second turns 62a, 62b in FIG. 3, are arranged to be in contact with one another. For reasons made clear below, in some embodiments the coiled electrode 26 includes at least one pair of adjacent turns 60, such as the turns 62c, 62d in FIG. 3, that are not in contact with one another, thereby defining a gap 70. The gap 70 can be formed during the winding or coiling process in shaping the wire(s) 60 as the coiled electrode 26. Regardless, the gap 70 is positioned more closely adjacent the proximal end 64 than the distal end 32, and is sized to permit passage of the tether line 28 therethrough. In other embodiments, however, the turns 62 are uniform in relation to one another along an entire length of the coiled electrode 26 such that the gap 70 is not provided or is otherwise eliminated.

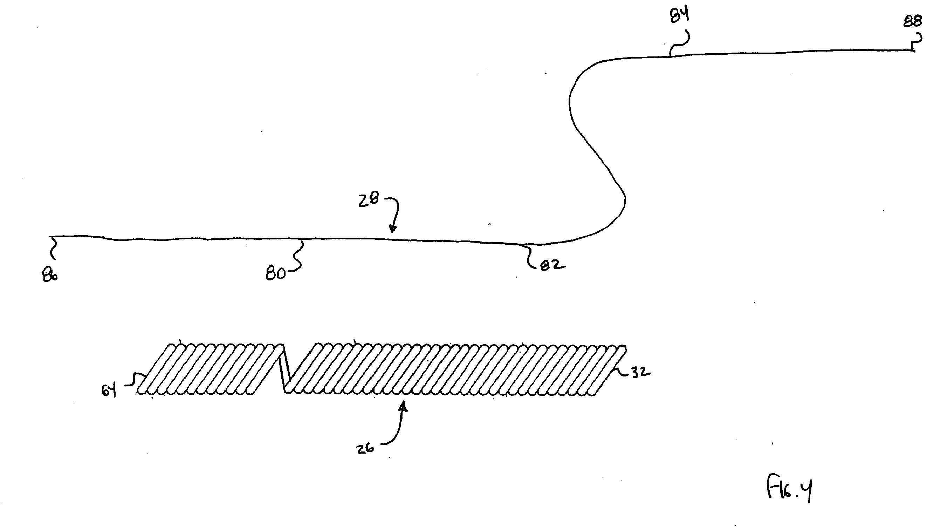

Returning to FIG. 1, the tether line 28 can assume a variety of forms, and is formed of a flexible material. For example, the tether line 28 can be a suture (e.g., silk suture, Prolene ™ suture, braided suture, etc.), although other material(s) or constructions are also acceptable (e.g., thread, fiber, wire, etc.). Regardless, and with additional reference to FIG. 4 that otherwise illustrates the tether line 28 prior to assembly to the coiled electrode 26, the tether line 28 can be defined as having a trailing segment 80, an intermediate segment 82, and a leading segment 84. The trailing segment 80 terminates at a trailing end 86, whereas the leading segment 84 terminates at a leading end 88.

With the above designations in mind, the tether line 28 is assembled to the coiled electrode 26 such that the trailing segment 80 is coupled to the electrode 26 at a point proximal the distal end 32, and the leading segment 84 extends from the coiled electrode 26 such that the leading end 88 is distal the distal end 32 as shown in FIGS. 5A and 5B. The tether line 28 passes through a portion of the central passageway 68, and is looped between an adjacent pair of the turns 62. The intermediate segment 82 extends within the central passageway 68 (best shown in FIG. 5B), and the trailing segment 80 is disposed along an exterior 100 (best shown in FIG. 5A) of the coiled electrode 26 , or the trailing segment 80 is disposed within the central passageway 68, with the intermediate segment 82 being positioned along the exterior 100. The trailing segment terminates at a trailing end, and the trailing end is bended to the coiled electrode proximal the distal end of the coiled electrode. Regardless, a transition 102 of the tether line 28 from the intermediate segment 82 to the trailing segment 80 passes or loops between an adjacent pair of the turns 62. In this manner, then tether line 28 effectively doubles back on itself over at least a partial length of the coiled electrode 26.

The above construction can be facilitated by provision of the gap 70. More particularly, the gap 70 provides a convenient location for passing of the tether line 28 between the adjacent turns 62c, 62d. With the one configuration above in which the tether line 28 is looped through the coiled electrode 26, the point at which the tether line 28 passes through the adjacent turns 62 (e.g., the turns 62c, 62d) effectively serves as the location point for transfer of a pulling force placed upon the tether line 28 to the coiled electrode 26. Thus, by proximally spacing the point at which the tether line 28 passes through the coiled electrode 26 from the distal end 32 thereof, the transferred force effectively serves to "push" the coiled electrode 26 distal the point of interface.

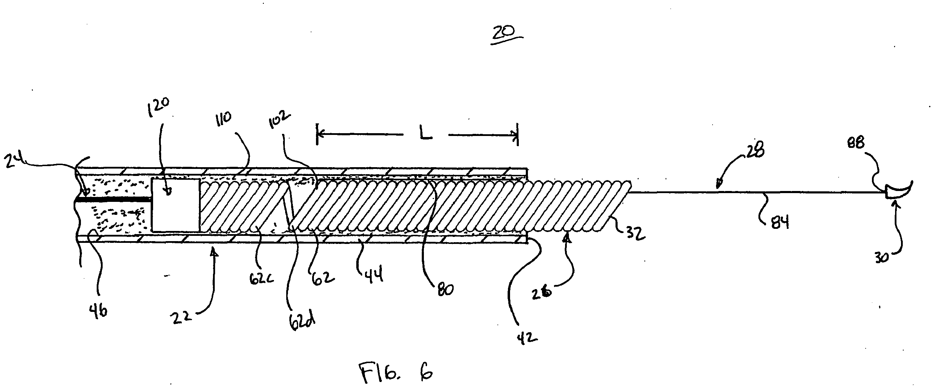

To promote a more complete affixment of the tether line 28 to the coiled electrode 26, an adhesive can be employed to bond the two components to one another. In this regard, and with additional reference to FIG. 6, the lead body 22 can be employed to effectuate a more complete connection. More particularly, and in some embodiments, the lumen 46 of the lead body 22 is sized to co-axially receive the coiled electrode 26, with a portion of the coiled electrode 26 extending distal the distal side 42 of the lead body 22. As shown, the coiled electrode 26/tether line 28 assembly is positioned related to the lead body 22 such that the trailing end 86 of the tether line 28 is adjacent the distal side 42 of the lead body 22 (or is not otherwise distally exposed relative to the distal side 42). Further, an adhesive 110 (referenced generally), such as a silicone adhesive, is disposed between the exterior 100 of the coiled electrode 26 and the side wall 44 of the lead body 22, and serves to affix or bond the tether line 28 relative to the coiled electrode 26 (as well as to the side wall 44 in some embodiments). The adhesive 110 can be positioned so as to subside at the distal side 42 of the lead body 22, as well as to encompass an entirety of the coiled electrode 26 within the lumen 46 (i.e., the portion of the coiled electrode 26 proximal the distal side 44).

To further assist in maintaining the lead assembly 20 upon assembly, a crimp ring 120 can be provided as shown in FIG. 6. The crimp ring 120 can be disposed within the lumen 46, and serves to secure the conductor 24 to the proximal end 64 (hidden in FIG. 6, but shown in FIG. 3) of the coiled electrode 26. As a point of reference, the adhesive 110 can be dispensed relative to the crimp ring 120 by inserting a delivery needle (not shown) through the lead body 22 and into the lumen 46 immediately proximal the crimp ring 120, with the adhesive 110, in flowable or solution form, then being dispensed through the needle. Regardless, with embodiments in which the crimp ring 120 and the adhesive 110 are provided, the adhesive can be positioned to encompass a longitudinal length on the order of 0.2 - 1.0 inch (5.08 - 25.4 mm) proximal the crimp ring 120 to better ensure a robust connection. Alternatively, the crimp ring 120 and/or the adhesive 110 can be omitted and are not required components, although some form of mechanical attachment (e.g., welding) between the coiled electrode 26 and the conductor 24 is desirable.

As reflected in FIG. 6, the trailing, segment 80 of the tether line 28 defines a suture overlap length L relative to the coiled electrode 26 upon final assembly. It has been surprisingly found that where the overlap length L is not less than 0.2 inch (5.08 mm), alternatively not less than 0.3 inch (7.62 mm), and alternatively not less than 0.37 inch (9.398 mm), the resultant assembly (in combination with adhesive bonding as described above) will maintain the affixment of the tether line 28 to the coiled electrode 26, and affixment of the coiled electrode 26 to the conductor 24 when the leading segment 84 of the tether line 28 is subjected to a pulling force of 0.50-lbf (2.22 N) (i.e., a force applied to the leading segment 84 in a distal direction). Alternatively, the lead assembly 20 can exhibit other affixment characteristics that can exceed or be less than the 0.50-lbf (2.22 N) pullout force parameter.

Finally, the needle 30, where provided, can be of any type known in the art, conventionally employed in piercing tissue, for example in delivering the coiled electrode 26 within target tissue. The needle 30 is coupled to the leading segment 84 of the tether line 28 (e.g., the leading end 88 is threaded through an opening of the needle 30 and tied), such that upon final assembly, the needle 30 is distal the distal end 32 of the coiled electrode 26.

Commensurate with the above, manufacture of the lead assembly 20 can include disposing the conductor 24 within the lead body 22, and coupling of the conductor 24 to the coiled electrode 26. The tether line 28 is passed within the central passageway 68 (FIG. 5B) of the coiled electrode 26, and looped between adjacent ones of the turns 62 at a point more closely adjacent the proximal end 64 (as compared to a location relative to the distal end 32). For example, the tether line 28 can be positioned such that the intermediate segment 82 (FIG. 5B) is within the central passageway 68 and the trailing segment 80 is along the exterior 100 of the coiled electrode 26. The tether line 28 is then affixed relative to the coiled electrode 26, such as by bonding the trailing segment 80 to the exterior 100 with the adhesive 110 as described above. In some embodiments, the adhesive 110 is not disposed within the central passageway 68 or does not otherwise effectuate bonding of the intermediate segment 82 to the coiled electrode 26 distal the point along the coiled electrode 26 at which the transition 102 passes between the adjacent turns 62 (e.g., the turns 62c, 62d). That is to say, while the trailing segment 80 can be bonded to the coiled electrode 26 distal the transition 102, the intermediate segment 82 is not; in this way, a pulling force applied to the leading segment 84 is transferred to, and imparted upon, the coiled electrode 26 at the transition 102. Were the intermediate segment 82 bonded to the coiled electrode 26 distal the transition 102, the distal most point of bonding between the intermediate segment 82 and the coiled electrode 26 would then serve as the point at which a pulling force applied to the leading segment 84 would be imparted upon the coiled electrode 26. It is desirable that the force transfer point be proximally spaced from the distal end 32 of the coiled electrode 26 as described above. In other embodiments, however, the intermediate segment 82 can be bonded or otherwise affixed to the coiled electrode 26 distal the transition 102.

In other embodiments, the lead assembly 20 can include additional features or components. For example, a cap having a distally tapering tip can be assembled over the coiled electrode 26 and associated with the distal side 42 of the lead body 22. Further, the lead assembly 20 can include one or more shaping features giving rise to enhanced flexibility and extensibility of the lead assembly 20 along a longitudinal length of the lead body 22 as described, for example, in U.S. Application Serial No. 11/413,316, filed April 28, 2006 and entitled "Implantable Medical Leads and Lead Assemblies With Improved Flexibility and Extensibility To Facilitate Body Movements" .

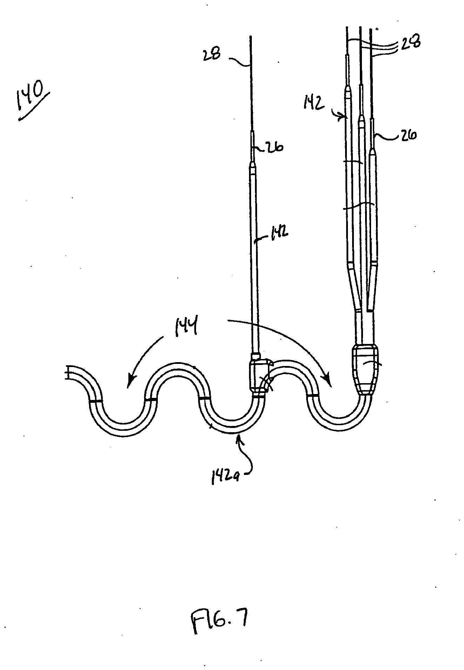

FIG. 7 illustrates one exemplary construction in accordance with this alternative embodiment, including a lead assembly 140 having a plurality of lead legs 142 at least one of which (e.g., the leg 142a) having a non-linear shape 144 (e.g., a sigmoid shape) that promotes longitudinal, elastic extension of the lead assembly 140.

Returning to FIGS. 1 and 5B, as part of an implantation procedure, the lead assembly 20 is provided as above, with the leading segment 84 of the tether line 28 (and the optional needle 30 assembled thereto) positioned or extending distal the distal end 32 of the coiled electrode 26. The lead body 22 is directed toward an implant target site (e.g., various bodily regions of a patient, such as a patient's neck, etc.). The needle 30 is manipulated by the surgeon to pierce through target tissue (e.g., muscle). A pulling force is applied to the needle 30 that in turn is transferred through the tether line 28 and on to the coiled electrode 26, sufficient to move the electrode 26 into contact with the target tissue. In this regard, the pulling force is focused upon the coiled electrode 26 at a point proximally spaced from the distal end 32. More particularly, the pulling force is transferred upon the point at which the transition 102 passes between adjacent turns 62, effectively transitioning the pulling force upon the needle 30 into a pushing force upon at least a majority of the coiled electrode 26. As a result, with continued pulling of the needle 30/tether line 28, the coiled electrode 26 is essentially pushed (i.e., that portion of the coiled electrode 26 distal the point at which the transition 102 passes between the adjacent turns 62 is "pushed") toward and into the target tissue.

Once the coiled electrode 26 has been desirably positioned or implanted within the target tissue, the tether line 28 is severed (e.g., at a point adjacent the distal end 32 of the coiled electrode 26), and the excess tether line 28 material and the needle 30 discarded. Once successfully implanted, the lead assembly 20 can be electrically connected to a stimulation generator (not shown) of a type known in the art, that can also be implanted within the patient at a point spaced from the implanted coiled electrode 26.

Although the present invention has been described with reference to preferred embodiments, workers skilled in the art will recognize that changes can be made in form and detail without departing from the scope of the present invention.

## Claims

1. An implantable medical electrical lead assembly comprising:
a lead body (22) maintaining an elongated conductor (24);
a coiled electrode (26) coupled to the conductor and defining a proximal end (64) and a distal end (32) relative to extension from the conductor; and
a tether line (28) defining a trailing segment (80) and a leading segment (84) terminating at a leading end (88), wherein the tether line is coupled to the coiled electrode proximal the distal end and the leading end extends distal the distal end of the coiled electrode; wherein
a pulling force applied to the leading segment of the tether line is transferred to the coiled electrode at a point proximal the distal end; and wherein the tether line further defines an intermediate segment between the leading and trailing segments, and further wherein a transition (102) of the intermediate segment to the trailing segment passes through adjacent turns of the coiled electrode; and wherein the coiled electrode defines a central passageway (68), and further wherein one of the intermediate segment and the trailing segment extends within the central passageway and the other of the intermediate segment and the trailing segment is disposed along an exterior of the coiled electrode; and wherein the trailing segment terminates at a trailing end, and further wherein the trailing end is bonded to the coiled electrode proximal the distal end; and wherein the coiled electrode includes a plurality of turns (62), and further wherein the plurality of turns forms a gap (70) at which first and second adjacent turns are not in contact with one another, the transition passing through the gap.

2. The lead assembly of claim 1, further comprising: a needle (30) attached to the leading segment of the tether line.

3. The lead assembly of claim 1, wherein the tether line is looped through the coiled electrode.

4. The lead assembly of claim 1, wherein the gap is formed more closely adjacent the proximal end (64) as compared to the distal end.

5. The lead assembly of claim 1, wherein the lead body includes a side wall (44) forming a lumen (46) along which a portion of the coiled electrode is disposed, the lead assembly further comprising: adhesive (110) bonding the tether line to the coiled electrode and the side wall.

6. The lead assembly of claim 5, wherein the lead body terminates at a distal side, and further wherein the adhesive is provided adjacent the distal side.

7. The lead assembly of claim 5, further comprising: a crimp ring (120) coupled to the proximal end of the coiled electrode and the conductor.

8. The lead assembly of claim 7, wherein the adhesive is provided along an entirety of a distal length of the lead body from the crimp ring to a distal side of the lead body.

9. The lead assembly of claim 1, wherein the trailing segment overlaps the coiled electrode over a length of not less than 0.37 inch (9.398 mm).

10. A method of making an implantable medical electrical lead assembly as claimed in any of claims 1 to 9, the method comprising:
providing a lead body (22) maintaining an elongated conductor (24);
providing a coiled electrode (26);
coupling the coiled electrode to the conductor such that the coiled electrode defines a proximal end and a distal end and a central passageway;
providing a tether line (28) defining a trailing segment and a leading segment terminating in a leading end, and an intermediate segment between the trailing segment and the leading segment and wherein a transition of the intermediate segment to the trailing segment passes through adjacent turns of the coiled electrode.;
coupling the tether line to the coiled electrode at a point proximally spaced from the distal end; and
extending the leading segment from the coiled electrode such that the leading end is distal the distal end; and further wherein one of the intermediate segment and the trailing segment extends within the central passageway and the other of the intermediate segment and the trailing segment is disposed along an exterior of the coiled electrode; and wherein the trailing segment terminates at a trailing end, and further wherein the trailing end is bonded to the coiled electrode proximal the distal end; and wherein the coiled electrode includes a plurality of turns (62), and further wherein the plurality of turns forms a gap (70) at which first and second adjacent turns are not in contact with one another, the transition passing through the gap

## Patentansprüche

1. Anordnung einer implantierbaren medizinischen elektrischen Leitung, die enthält:
einen Leitungskörper (22), der einen lang gestreckten Leiter (24) hält;
eine gewickelte Elektrode (26), die mit dem Leiter gekoppelt ist und in Bezug auf die Erstreckung des Leiters ein proximales Ende (64) und ein distales Ende (32) definiert; und
eine Halteleine (28), die ein hinteres Segment (80) und ein vorderes Segment (84), das an einem vorderen Ende (88) endet, definiert, wobei die Halteleine mit der gewickelten Elektrode proximal des distalen Endes gekoppelt ist und das vordere Ende sich distal bezüglich des distalen Endes der gewickelten Elektrode erstreckt; wobei
eine Zugkraft, die auf das vordere Segment der Halteleine ausgeübt wird, an die gewickelte Elektrode an einem Punkt proximal des distalen Endes übertragen wird; und wobei die Halteleine ferner ein Zwischensegment zwischen dem vorderen und dem hinteren Segment definiert und wobei ferner ein Übergang (102) des Zwischensegments zu dem hinteren Segment durch benachbarte Windungen der gewickelten Elektrode verläuft; und wobei die gewickelte Elektrode einen mittigen Durchlassweg (68) definiert und wobei sich ferner das Zwischensegment oder das hintere Segment in dem mittigen Durchlassweg erstreckt und das jeweils andere des Zwischensegments und des hinteren Segments längs einer Außenseite der gewickelten Elektrode angeordnet ist; und wobei das hintere Segment an einem hinteren Ende endet und wobei ferner das hintere Ende an der gewickelten Elektrode proximal des distalen Endes haftet; und wobei die gewickelte Elektrode mehrere Windungen (62) aufweist und wobei ferner die mehreren Windungen einen Spalt (70) bilden, bei dem erste und zweite benachbarte Windungen nicht miteinander in Kontakt sind, wobei der Übergang durch den Spalt verläuft.

2. Leitungsanordnung nach Anspruch 1, ferner mit einer Nadel (30), die an dem vorderen Segment der Halteleine befestigt ist.

3. Leitungsanordnung nach Anspruch 1, wobei die Halteleine durch die gewickelte Elektrode geschleift ist.

4. Leitungsanordnung nach Anspruch 1, wobei der Spalt im Vergleich zu dem distalen Ende näher bei dem proximalen Ende (64) ausgebildet ist.

5. Leitungsanordnung (1) nach Anspruch 1, wobei der Leitungskörper eine Seitenwand (44), die ein Lumen (46) bildet, aufweist, längs derer ein Abschnitt der gewickelten Elektrode angeordnet ist, wobei die Leitungsanordnung ferner Klebstoff (110) aufweist, mit dem die Halteleine an der gewickelten Elektrode und an der Seitenwand haftet.

6. Leitungsanordnung nach Anspruch 5, wobei der Leitungskörper an einem distalen Ende endet und wobei ferner der Klebstoff neben der distalen Seite vorgesehen ist.

7. Leitungsanordnung nach Anspruch 5, ferner mit einem Falzring (120), der mit dem proximalen Ende der gewickelten Elektrode und mit dem Leiter gekoppelt ist.

8. Leitungsanordnung nach Anspruch 7, wobei der Klebstoff längs der gesamten distalen Länge des Leitungskörpers von dem Falzring zu einer distalen Seite des Leitungskörpers vorgesehen ist.

9. Leitungsanordnung nach Anspruch 1, wobei das hintere Ende mit der gewickelten Elektrode über eine Länge von nicht weniger als 0,37 Zoll (9,398 mm) überlappt.

10. Verfahren zum Herstellen einer Anordnung einer implantierbaren medizinischen elektrischen Leitung nach einem der Ansprüche 1 bis 9, wobei das Verfahren umfasst:
Vorsehen eines Leitungskörpers (22), der einen lang gestreckten Leiter (24) hält;
Vorsehen einer gewickelten Elektrode (26);
Koppeln der gewickelten Elektrode mit dem Leiter in der Weise, dass die gewickelte Elektrode ein proximales Ende und ein distales Ende sowie einen mittigen Durchlassweg definiert;
Vorsehen einer Halteleine (28), die ein hinteres Segment und ein vorderes Segment, das in einem vorderen Ende endet, und ein Zwischensegment zwischen dem hinteren Segment und dem vorderen Segment definiert, wobei ein Übergang des Zwischensegments zu dem hinteren Segment durch benachbarte Windungen der gewickelten Elektrode verläuft;
Koppeln der Halteleine mit der gewickelten Elektrode an einem Punkt, der von dem distalen Ende proximal beabstandet ist; und
Verlängern des vorderen Segments von der gewickelten Elektrode in der Weise, dass das vordere Ende zu dem distalen Ende distal ist; wobei sich ferner das Zwischensegment oder das hintere Segment in dem mittigen Durchlassweg erstreckt und das jeweils andere des Zwischensegments und des hinteren Segments längs einer Außenseite der gewickelten Elektrode angeordnet ist; wobei das hintere Segment an einem hinteren Ende endet und wobei ferner das hintere Ende an der gewickelten Elektrode proximal zu dem distalen Ende haftet; und wobei die gewickelte Elektrode mehrere Windungen (62) aufweist und wobei ferner die mehreren Windungen einen Spalt (70) bilden, bei dem erste und zweite benachbarte Windungen nicht miteinander in Kontakt sind, wobei der Übergang durch den Spalt verläuft.

## Revendications

1. Ensemble de fil électrique médical implantable comportant :
un corps de fil (22) maintenant un conducteur allongé (24) ;
une électrode en spirale (26) couplée au conducteur et définissant une extrémité proximale (64) et une extrémité distale (32) par rapport à une extension à partir du conducteur ; et
une ligne d'attache (28) définissant un segment arrière (80) et un segment avant (84) se terminant sur une extrémité avant (88), dans lequel la ligne d'attache est couplée à l'électrode en spirale proximale à l'extrémité distale et
l'extrémité avant s'étend distale à l'extrémité distale de l'électrode en spirale ; dans lequel
une force de traction appliquée au segment avant de la ligne d'attache est transférée à l'électrode en spirale en un point proximal à l'extrémité distale ; et dans lequel la ligne d'attache définit en outre un segment intermédiaire entre les segments avant et arrière, et également dans lequel une transition (102) du segment intermédiaire jusqu'au segment arrière passe à travers des spires adjacentes de l'électrode en spirale ; et dans lequel l'électrode en spirale définit un passage central (68), et également dans lequel un segment parmi le segment intermédiaire et le segment arrière s'étend à l'intérieur du passage central et
l'autre segment parmi le segment intermédiaire et le segment arrière est disposé le long d'un extérieur de l'électrode en spirale ; et dans lequel le segment arrière se termine sur une extrémité arrière, et également dans lequel l'extrémité arrière est assemblée à l'électrode en spirale proximale à l'extrémité distale ; et dans lequel l'électrode en spirale inclut une pluralité de spires (62),
et également dans lequel la pluralité de spires forme un espace (70) sur lequel des première et seconde spires adjacentes ne sont pas en contact l'une avec l'autre, la transition passant à travers l'espace.

2. Ensemble de fil selon la revendication 1, comportant également : une aiguille (30) attachée au segment avant de la ligne d'attache.

3. Ensemble de fil selon la revendication 1, dans lequel la ligne d'attache est bouclée à travers l'électrode en spirale.

4. Ensemble de fil selon la revendication 1, dans lequel l'espace est formé de manière plus étroitement adjacente à l'extrémité proximale (64) que par rapport à l'extrémité distale.

5. Ensemble de fil selon la revendication 1, dans lequel le corps de fil inclut une paroi latérale (44) formant une lumière (46) le long de laquelle une partie de l'électrode en spirale est disposée, l'ensemble de fil comportant en outre : un adhésif (110) assemblant la ligne d'attache à l'électrode en spirale et à la paroi latérale.

6. Ensemble de fil selon la revendication 5, dans lequel le corps de fil se termine sur un côté distal, et également dans lequel l'adhésif est agencé adjacent au côté distal.

7. Ensemble de fil selon la revendication 5, comportant en outre : une bague de sertissage (120) couplée à l'extrémité proximale de l'électrode en spirale et du conducteur.

8. Ensemble de fil selon la revendication 7, dans lequel l'adhésif est agencé le long de la totalité d'une longueur distale du corps de fil depuis la bague de sertissage jusqu'à un côté distal du corps de fil.

9. Ensemble de fil selon la revendication 1, dans lequel le segment arrière chevauche l'électrode en spirale sur une longueur non inférieure à 9,398 mm (0,37 pouce).

10. Procédé de fabrication d'un ensemble de fil électrique médical implantable tel que revendiqué dans l'une quelconque des revendications 1 à 9, le procédé comportant les étapes consistant à :
fournir un corps de fil (22) maintenant un conducteur allongé (24) ;
fournir une électrode en spirale (26);
coupler l'électrode en spirale au conducteur de telle sorte que l'électrode en spirale définit une extrémité proximale et une extrémité distale et un passage central ;
fournir une ligne d'attache (28) définissant un segment arrière et un segment avant se terminant dans une extrémité avant, et un segment intermédiaire entre le segment arrière et le segment avant et dans lequel une transition du segment intermédiaire jusqu'au segment arrière passe à travers des spires adjacentes de l'électrode en spirale ;
coupler la ligne d'attache à l'électrode en spirale en un point espacé de manière proximale par rapport à l'extrémité distale ; et
étendre le segment avant à partir de l'électrode en spirale de telle sorte que l'extrémité avant est distale à l'extrémité distale ; et également dans lequel un segment parmi le segment intermédiaire et le segment arrière s'étend à l'intérieur du passage central et l'autre segment parmi le segment intermédiaire et le segment arrière est disposé le long d'un extérieur de l'électrode en spirale ;
et dans lequel le segment arrière se termine sur une extrémité arrière, et également dans lequel l'extrémité arrière est assemblée à l'électrode en spirale proximale à l'extrémité distale ; et dans lequel l'électrode en spirale inclut une pluralité de spires (62), et également dans lequel la pluralité de spires forme un espace (70) sur lequel des première et seconde spires adjacentes ne sont pas en contact l'une avec l'autre, la transition passant à travers l'espace.
